# EUROPÄISCHE PATENTSCHRIFT

(11) **EP 1 086 047 B1**
(45) Veröffentlichungstag und Bekanntmachung des Hinweises auf die Patenterteilung: **03.12.2003**
(21) Anmeldenummer: 99903615.5
(22) Anmeldetag: 09.01.1999
(51) Int. Cl.: C02F 1/32, E03C 1/04

(54) **SANITÄRARMATUR**
SANITARY APPLIANCY
ROBINETTERIE SANITAIRE

(30) Priorität: 27.03.1998 DE 19813544
(43) Veröffentlichungstag der Anmeldung: 28.03.2001
(73) Patentinhaber: HANSA METALLWERKE AG, 70567 Stuttgart (DE)
(72) Erfinder: KUNKEL, Horst, D-70499 Stuttgart (DE)
(74) Vertreter: Ostertag, Reinhard
(86) Internationale Anmeldenummer: EP9900092
(87) Internationale Veröffentlichungsnummer: WO99050183

(56) Entgegenhaltungen:
- EP-A- 0 769 322
- WO-A-95/07860
- WO-A-98/17390
- DE-A- 4 440 880
- DE-A- 19 626 134
- US-A- 5 124 131
- US-A- 5 141 636
- US-A- 5 466 425

## Beschreibung

Die Erfindung betrifft eine Sanitärarmatur.

In den letzten Jahren sind die Gefahren, die von mit Mikroorganismen, insbesondere Bakterien, Amöben oder anderen Einzellern, verunreinigtem Wasser im Sanitärbereich ausgehen, zunehmend deutlicher geworden. Der Entkeimung von Wasser wird daher eine erhöhte Bedeutung beigemessen. In diesem Zusammenhang wurde von Versuchen berichtet, strömendes Wasser durch Bestrahlung mit UV-Licht zu entkeimen, dessen Längenwelle geeignet ist, im Wasser mitgeführte Mikroorganismen abzutöten. Zur Verzögerung der Verweilzeit dieser mikroorganismen im Bereich der UV-Lampe kann eine zusätzliche Filtervorrichtung eingesetzt werden.

In der US 51 24 131 A ist eine Entkeimungseinrichtung beschrieben, die zur Entkeimung größerer Wassermengen, wie sie beispielsweise bei Industrieanlagen anfallen, konzipiert ist. Innerhalb eines Gehäuses liegen mehrere UV-Lampen vor. Das zu entkeimende Wasser strömt durch das Gehäuse an den parallel zueinander angeordneten UV-Lampen vorbei. Die inneren Seitenwände des Gehäuses sind optional mit einer UV-reflektierenden Schicht versehen. Als Beispiel für eine derartige Schicht wird Aluminium, das mit Magnesiumfluorid beschichtet ist, angegeben. Die UV-reflektierende Schicht ist ihrerseits mit einer Teflon-Schicht versehen, wodurch eine Auflösung der UV-reflektierenden Schicht durch das vorbeiströmende Wasser vermieden werden soll. Der Einsatz der Wasserentkeimungsrichtung direkt in einer Sanitäreinrichtung wird hier nicht beschrieben.

In der DE 44 40 880 A1 ist eine Entkeimungseinrichtung für Abwässer beschrieben, die industriell anfallen. Bei einer Ausführungsform bildet sich an der Innenseite eines hohlzylinderförmigen Gehäuses durch Rotation um dessen zentrale Achse eine dünne Schicht von Abwasser. Diese wird von einer UV-Lampe bestrahlt. Die Innenseite des Gehäuses ist mit einer UV-reflektierenden Schicht versehen, die wiederum mit Teflon beschichtet ist. Diese Einrichtung ist zur Entkeimung stark kontaminierter Wässer konzipiert; die Entkeimung direkt in der Sanitäreinrichtung ist nicht angesprochen.

In der US 54 66 425 A ist eine Wasserentkeimungseinrichtung beschrieben, beschrieben wird, die sowohl zur Entkeimung von Industrieanlagen- und Stadtabwässern als auch zur endverbrauchernahen Trinkwasserentkeimung dienen kann. In einem Ausführungsbeispiel weist sie ein hohlzylinderförmiges Gehäuse mit einer zentral angeordneten UV-Lampe auf, zwischen denen ein Durchströmungsraum für das zu entkeimende Wasser verbleibt. Die Innenwand des Gehäuses ist mit einer UV reflektierenden Beschichtung überzogen. Für die Beschichtung wiederum ist eine Schutzschicht vorgesehen, die eine Dotierung aufweist, die UV-Strahlung absorbiert und Wellenlängen verschoben reemittiert. Auch diese Entkeimungseinrichtung steht für sich alleine und befindet sich nicht direkt in einer Sanitäreinrichtung.

Die US 51 41 636 A offenbart schließlich ein Ausführungsbeispiel einer Entkeimungeinrichtung mit einer zylinderförmigen UV-Lampe in einem Gehäuse, die von einer ebenfalls im Gehäuse angeordneten wendelförmigen Rohrleitung umgeben ist. Durch diese strömendes Wasser wird mit dem Licht der UV-Lampe behandelt, da die Rohrleitung aus Teflon für bestimmte Wellenlängen durchlässig ist. Zur Erhöhung der Lampeneffizienz ist das Gehäuse mit einer reflektierenden inneren Oberfläche ausgestattet. Erneut handelt es sich hier um eine separate Entkeimungseinrichtung, die sich nicht direkt in einer Sanitärarmatur befindet.

Aufgabe der vorliegenden Erfindung ist es, eine Sanitäreinrichtung der eingangs genannten Art derart auszugestalten, daß das sie durchströmende Wasser direkt in ihr entkeimt wird.

Diese Aufgabe wird durch die im Anspruch 1 angegebene Erfindung gelöst.

Erfindungsgemäß wird direkt in der Sanitäreinrichtung eine UV-Lampe angeordnet, deren Strahlung auf das durchströmende Wasser und ggfs. auf eine ebenfalls in dem Gehäuse untergebrachte Filtervorrichtung gerichtet ist. Zur Erhöhung der Effizienz wird ein zusätzlicher Reflektor eingesetzt, der die äußere Begrenzung des vom zu desinfizierenden Wasser durchströmten Raumes ist. Die innere Begrenzung dieses Wasserweges wird dann vom Leuchtkörper der UV-Lampe bzw. von einem diesen Leuchtkörper umgebenden Schutzrohr gebildet. Das von der UV-Lampe ausgehende UV-Licht wird nicht bereits nach dem ersten Durchgang durch den vom Wasser durchströmten Raum und eine hier ggfs. vorgesehene Filtervorrichtung von dem Gehäuse im wesentlichen absorbiert, sondern wird, entsprechend dem Reflexionsvermögen des Reflektors, vielfach hin- und herreflektiert, so daß die für die Desinfektion wirksame Lichtintensität innerhalb des vom Reflektor umgebenen Raumes erheblich vergrößert ist.

Bei einer besonders bevorzugten Ausführungsform der Erfindung weist der Reflektor eine der UV-Lampe zugewandte Schicht aus für UV-Licht transparentem Material, welches gegenüber Wasser korrosionsbeständig und in hygienischer Richtung unbedenklich ist, und eine von der UV-Lampe abgewandte Schicht mit hohem Reflexionsvermögen für UV-Strahlung auf. Mit dieser Ausgestaltung der Erfindung wird der Tatsache Rechnung getragen, daß Substanzen, die ein hohes Reflexionsvermögen für UVC-Licht aufweisen, im allgemeinen gegenüber Wasser nicht beständig sind und aus gesundheitlichen Gründen nicht in direkte Berührung mit dem strömenden Wasser gelangen dürfen. Durch den zweischichtigen Aufbau wird dieses Problem beseitigt: Die nach innen, auf die UV-Lampe zu zeigende Schicht aus transparentem Material kann so gewählt werden, daß sie gegen das strömende Wasser beständig ist und außerdem allen hygienischen Anforderungen im Frischwasserbereich genügt.

Die hochreflektierende Schicht besteht bevorzugt aus Aluminium oder aus MnO.

Was die strahlungsdurchlässige Schicht angeht, so wird bevorzugt, daß diese aus PTFE oder TiO₂ besteht.

Besonders geeignet scheint insbesondere diejenige Ausgestaltung der Erfindung, bei welcher die strahlungsdurchlässige Schicht aus einem anorganisch-organischen Hybridpolymer, vorzugsweise mit einem Silikatnetzwerk als anorganischer Komponente, besteht. Derartige Hybridpolymere weisen nicht nur besonders gute und in ihren Eigenschaften gezielt herstellbare Barriereneigenschaften auf; sie sind auch in ihrer optischen Durchlässigkeit durch Wahl der Strukturelemente beeinflußbar und können daher für den vorliegndden Verwendungszweck maßgeschneidert werden.

Ein Ausführungsbeispiel der Erfindung wird nachfolgend anhand der Zeichnung näher erläutert; es zeigen:
- Figur 1: einen Wannenauslauf, der mit einer Einrichtung zur Entkeimung und Filtrierung von durchlaufendem Wasser versehen ist;
- Figur 2: einen schematischen Axialschnitt durch einen Teil des in den Wannenauslauf von Figur 1 eingesetzten Reflektors.

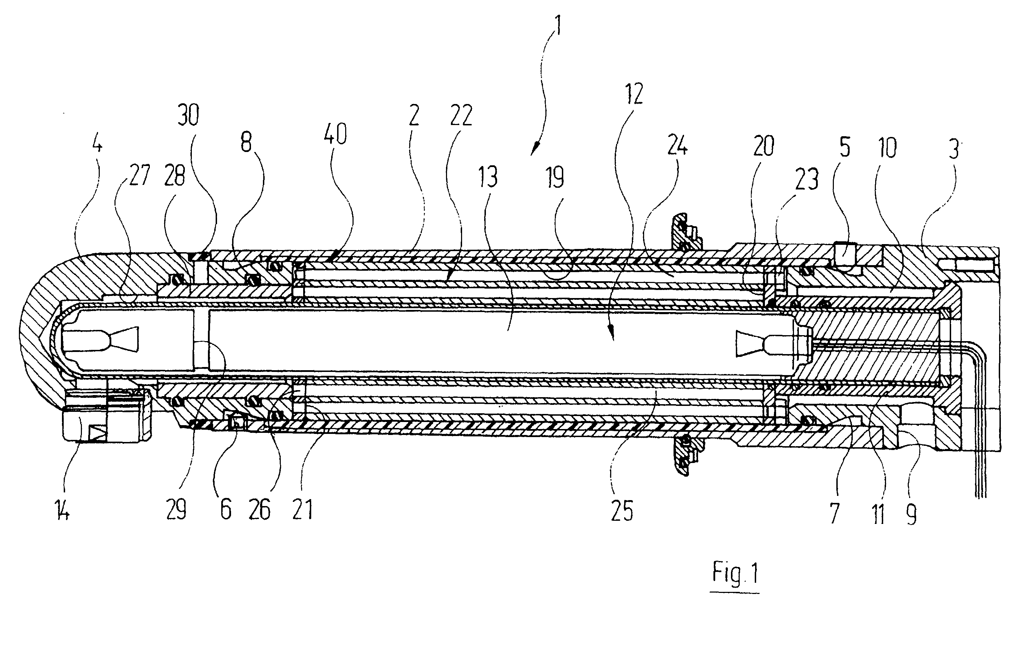

Der Wannenauslauf, der insgesamt durch das Bezugszeichen 1 gekennzeichnet ist, umfaßt einen im wesentlichen hohlzylindrischen Gehäusemantel 2, der an seinem in der Figur 1 rechten, der Gebäudewand benachbarten Ende durch einen Montagesockel 3 und an seinem gegenüberliegenden Ende durch einen Auslaufkopf 4 verschlossen ist. Montagesockel 3 und Auslaufkopf 4 sind jeweils durch eine den Gehäusemantel 2 durchdringende Madenschraube 5 bzw. 6 an dem Gehäusemantel 2 montiert, welche in eine Umfangsnut 7 bzw. 8 an dem Montagesockel 3 bzw. dem Auslaufkopf 4 eingreift.

Der Montagesockel 3 weist eine parallel zur nicht dargestellten Montagewand verlaufende Wasserzulauföffnung 9 auf, die in einen Wasserzulaufraum 10 einmündet. Der Wasserzulaufraum 10 umgibt ringförmig einen Aufnahmesockel 11 für eine UV-Lampe 12, deren Leuchtkörper 13 sich axial durch den gesamten Gehäusemantel 2 hindurch bis in den Auslaufkopf 4 und dort bis über den in diesem vorgesehenen, nach unten zeigenden, als Auslauföffnung dienenden Luftsprudler 14.

Innerhalb des zylindrischen Gehäusemantels 2 ist ein Behandlungsraum 19 ausgebildet, der an beiden Stirnseiten jeweils durch eine Zwischenplatte 20 bzw. 21 begrenzt ist. In dem Behandlungsraum 19 ist eine Filtervorrichtung 22 untergebracht, welche den Leuchtkörper 13 der UV-Lampe 12 ringförmig umgibt. Durchtrittsöffnungen 23 in der ersten Zwischenplatte 20 sorgen dafür, daß das über die Zulauföffnung 9 in den Wasserzulaufraum 10 zuströmende Wasser in einen ersten Teilraum 24 der Filtervorrichtung 22 strömt. Aus diesem durchtritt das Wasser einen filteraktiven Bereich der Filtervorrichtung 22 und gelangt in einen zweiten Teilraum 25, von wo es über Durchgangsöffnungen 26 der zweiten Zwischenplatte 21 in einen ringförmigen Auslaufraum 27 innerhalb des Auslaufkopfes 4 gelangt, welcher mit dem Luftsprudler 14 kommuniziert. Einzelheiten der Ausgestaltung der Filtervorrichtung 22 und der Wasserführung zwischen der Zulauföffnung 9 und dem Luftsprudler 14 sind hier nicht von Interesse.

In eine Erweiterung des nach rechts in der Zeichnung offenen Auslaufkopfes 4 ist ein Glasrohrstück 28 eingeschoben, welches den Leuchtkörper 13 der UV-Lampe 12 auf einem Stück ihrer axialen Länge umgibt. Es besteht aus einem Material, welches für UVC-Strahlung undurchlässig ist, und ist durch O-Ringe in der dargestellten Weise gegen den Auslaufkopf 4 abgedichtet.

In demjenigen axialen Bereich, in welchem sich das Glasrohrstück 28 befindet, ist der Auslaufkopf 4 mit einem ringsegmentartigen Fenster 29 versehen. Über diesem Fenster 29 ist ein Ring 30 aus einem transparenten, nicht notwendig klaren aber fluoreszierendem Kunststoffmaterial eingelegt. Dieser Ring 30 übernimmt gleichzeitig die Aufgaben einer Funktionsanzeige und einer Abdichtung zwischen dem hohlzylindrischen Gehäusemantel 2 und dem Auslaufkopf 4.

In eine Ausnehmung an der Innenfläche des Gehäusemantels 2 ist ein rohrförmiger Reflektor 40 eingesetzt, der die äußere Begrenzung des Behandlungsraumes 19 bildet. Die Anordnung ist so, daß das Wasser zwischen der UV-Lampe 12 und dem hierzu koaxialen Reflektor 40 strömt.

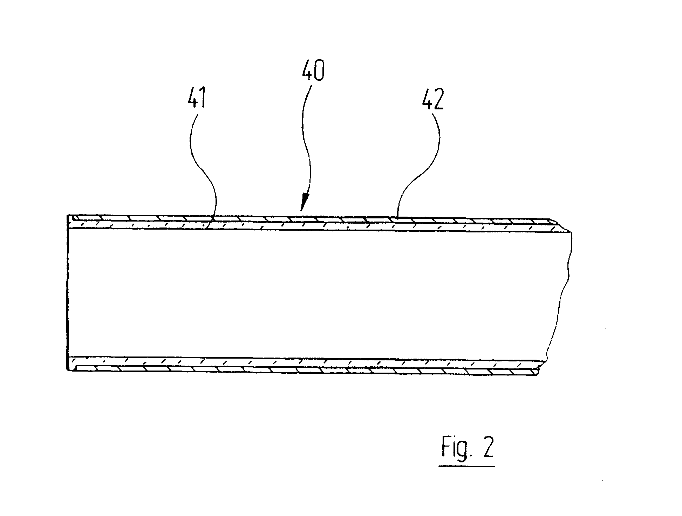

Der genaue Aufbau des Relektors 40 ist der Figur 2 zu entnehmen. Er umfaßt eine radial außenliegene Schicht 42 aus einem Material, welches ein hohes Reflexionsvermögen für UVC-Strahlung aufweist, die für die Desinfektionswirkung von besonderer Bedeutung ist. An die Schicht 42 ist radial nach innen und außerdem stirnseitig eine weitere Schicht 41 angefügt, die für die angesprochene UVC-Strahlung durchlässig ist, gleichzeitig aber gegenüber dem Wasser, welches innerhalb der Schicht 41 des Reflektors 40 strömt, beständig und für den Einsatz im Frischwasserbereich in hygienischer Hinsicht zugelassen ist.

Die Figur 2 kann beispielsweise wie folgt gelesen werden: Die radial außenliegende Schicht 42 ist ein hochpoliertes Aluminiumrohr, welches an der Innenmantelfläche und stirnseitig mit PTFE, welches die Schicht 41 bildet, ausgekleidet ist.

Die Figur 2 läßt sich auch in folgender Weise verstehen: Das Bezugszeichen 41 bezeichnet ein Rohr aus PTFE, welches an der Außenmantelfläche mit einer Schicht (Bezugszeichen 42) aus hochreflektierendem MnO überzogen ist.

Als Material für die strahlungsdurchlässige Schicht 41 von Figur 2 eignen sich insbesondere auch anorganisch-organische Mischpolymere, wie sie unter der Marke "Ormocere" bekannt geworden sind. Es handelt sich dabei um die Ergebnisse von Sol-Gel-Reaktionen organisch modifizierter Si-Alkoxide: In einem ersten Schritt wird ein anorganisches Silikat-Netzwerk aufgebaut, wobei eine Kokondensation mit anderen Metalloxiden möglich ist. In einem zweiten Schritt erfolgt dann unter dem Einfluß von Wärme oder Strahlung der Aufbau eines organischen Netzwerkes, etwa auf der Basis von Acryl, Vinyl. Epoxy und ähnlichen Substanzen.

Derartige Hybridpolymere lassen sich durch Wahl der Bestandteile und die Reaktionsbedingungen sowohl hinsichtlich ihrer Barriereeigenschaften als auch ihrer Strahlungsdurchlässigkeit optimal an den vorliegenden Verwendungszweck anpassen.

Die Funktion der beschriebenen Einrichtung ist wie folgt:

Wird ein in der Zeichnung nicht dargestelltes Ventil geöffnet, so strömt Wasser über die Zulauföffnung 9 und den Wasserzulaufraum 10 in den Behandlungsraum 19 ein. Zu diesem Zeitpunkt ist in hier nicht interessierender Weise die UV-Lampe 12 bereits eingeschaltet. Das Wasser, welches über den ersten Teilraum 24 der Filtervorrichtung 22, den filteraktiven Bereich der Filtervorrichtung 22, den zweiten Teilraum 25 und über die Durchgangsöffnungen 26 der zweiten Zwischenplatte 21 zum Auslaufraum 27 und von dort zum Luftsprudler 14 fließt, wird also von den Strahlen, welche die UV-Lampe 12 aussendet, erreicht. Hierin befindliche Mikroorganismen werden abgetötet. Beim Durchströmen der Filtervorrichtung 22 werden diese Mikroorganismen außerdem zurückgehalten.

Das von der UV-Lampe 12 ausgehenden UVC-Licht wird von dem Reflektor 40, entsprechend dem Reflexionsvermögen der in ihm enthaltenen Schicht 42, mehrfach hin- und herreflektiert, so daß sich in dem vom Reflektor 40 umgebenen Raum, der vom zu reinigenden Wasser durchströmt ist und in dem sich auch die Filtervorrichtung 22 befindet, eine hohe Intensität der UVC-Strahlung aufbaut. Dies ermöglicht eine zuverlässige Desinfektion des zuströmenden Wassers bzw. die Abtötung von Mikroorganismen, die von der Filtervorrichtung 22 zurückgehalten werden.

Muß die Filtervorrichtung 22 oder der Reflektor 40 ausgewechselt werden, kann der Auslaufkopf 4 einfach nach Lösen der Madenschraube 6 von dem Gehäusemantel 2 abgezogen werden, worauf die zweite Zwischenplatte 21 freiliegt. Diese kann jetzt aus dem Gehäusemantel 2 herausgezogen werden. Nun ist die Filtervorrichtung 22 oder der Reflektor 40 zum Auswechseln zugänglich. Die Wiedermontage der verschiedenen Teile erfolgt in umgekehrter Reihenfolge.

## Patentansprüche

1. Sanitärarmatur mit
a) einem Gehäuse (2), welches einen Einlaß (9) und einen Auslaß (4) für das Wasser aufweist;
b) einer UV-Lampe (12), die innerhalb des Gehäuses (2) angeordnet und deren Strahlung auf das durchströmende Wasser und ggfs. auf eine ebenfalls in dem Gehäuse (2) untergebrachte Filtervorrichtung (22) gerichtet ist;
c) einem Reflektor (40), der an der Innenfläche des Gehäuses (2) angeordnet ist und den Leuchtkörper (13) der UV-Lampe (12) zumindest bereichsweise derart umgibt, daß das Wasser den Zwischenraum zwischen dem Leuchtkörper (13) der UV-Lampe (12) und dem Refklektor (40) durchströmt.

2. Sanitärarmatur nach Anspruch 1, **dadurch gekennzeichnet, daß** der Reflektor (40) eine der UV-Lampe (12) zugewandte Schicht (41) aus einem UV-Licht-transparenten Material, welches gegenüber Wasser korrosionsbeständig und in hygienischer Richtung unbedenklich ist, und eine von der UV-Lampe (12) abgewandte Schicht (42) mit hohem Reflexionsvermögen für UV-Strahlung aufweist.

3. Sanitärarmatur nach Anspruch 2, **dadurch gekennzeichnet, daß** die hoch reflektive Schicht (42) aus Aluminium besteht.

4. Sanitärarmatur nach Anspruch 2, **dadurch gekennzeichnet, daß** die hoch reflektive Schicht (42) aus MnO besteht.

5. Sanitärarmatur nach einem der Ansprüche 2 bis 4, **dadurch gekennzeichnet, daß** die strahlungsdurchlässige Schicht (41) aus PTFE besteht.

6. Sanitärarmatur nach einem der Ansprüche 2 bis 4, **dadurch gekennzeichnet, daß** die strahlungsdurchlässige Schicht (41) aus TiO₂ besteht.

7. Sanitärarmatur nach einem der Ansprüche 2 bis 4, **dadurch gekennzeichnet, daß** die strahlungsdurchlässige Schicht (41) aus einem anorganisch-organischen Hybridpolymer besteht.

8. Sanitärarmatur nach Anspruch 7, **dadurch gekennzeichnet, daß** das anorganisch-organische Hybridpolymer als anorganische Komponente ein Silikatnetzwerk enthält.

## Claims

1. Sanitary fitting having
a) a housing (2) which has an inlet (9) and an outlet (4) for water,
b) a UV lamp (12) which is arranged inside the housing (2) and whose radiation is directed onto the water flowing through and, where applicable, onto a filter device (22) which is also accommodated in the housing (2),
c) a reflector (40) which is arranged on the inside surface of the housing (2) and which surrounds the luminescing body (13) of the UV lamp (12), at least regionally, in such a way that the water flows through the gap between the luminescing body (13) of the UV lamp (12) and the reflector (40).

2. Sanitary fitting according to claim 1, **characterised in that** the reflector (40) has a layer (41), adjacent the UV lamp (12), of a material transparent to UV light which is resistant to corrosion by water and is unobjectionable from a hygienic point of view, and a layer (42) remote from the UV lamp (12) having a high reflectivity for UV radiation.

3. Sanitary fitting according to claim 2, **characterised in that** the highly reflective layer (42) is composed of aluminium.

4. Sanitary fitting according to claim 2, **characterised in that** the highly reflective layer (42) is composed of MnO.

5. Sanitary fitting according to one of claims 2 to 4, **characterised in that** the layer (41) permeable to radiation is composed of PTFE.

6. Sanitary fitting according to one of claims 2 to 4, **characterised in that** the layer (41) permeable to radiation is composed of TiO₂.

7. Sanitary fitting according to one of claims 2 to 4, **characterised in that** the layer (41) permeable to radiation is composed of an inorganic/organic hybrid polymer.

8. Sanitary fitting according to claim 7, **characterised in that** the inorganic/organic hybrid polymer contains a silicate network as an inorganic component.

## Revendications

1. Robinetterie sanitaire avec
a) un boîtier (2) qui comporte une admission (9) et une sortie (4) pour l'eau ;
b) une lampe à UV (12) disposée à l'intérieur du boîtier (2) et dont le rayonnement est dirigé vers l'écoulement d'eau et, le cas échéant, sur un dispositif de filtration (22) également disposé dans le boîtier (2) ;
c) un réflecteur (40) disposé sur la face intérieure du boîtier (2) et qui entoure au moins partiellement l'élément lumineux (13) de la lampe à UV (12) de telle sorte que l'eau s'écoule dans l'espace intermédiaire entre l'élément lumineux (13) de la lampe à UV (12) et le réflecteur (40).

2. Robinetterie sanitaire selon la revendication 1, **caractérisée en ce que** le réflecteur (40) comporte une couche (41) de matériau laissant passer la lumière UV, résistant à la corrosion dans l'eau et neutre du point de vue hygiénique, orientée vers la lampe UV (12), et une couche (42) orientée du côté opposé à la lampe UV (12) avec un fort pouvoir de réflexion des rayons UV.

3. Robinetterie sanitaire selon la revendication 2, **caractérisée en ce que** la couche à fort pouvoir réfléchissant (42) est constituée d'aluminium.

4. Robinetterie sanitaire selon la revendication 2, **caractérisée en ce que** la couche à fort pouvoir réfléchissant (42) est constituée de MnO.

5. Robinetterie sanitaire selon l'une des revendications 2 à 4, **caractérisée en ce que** la couche laissant passer le rayonnement (41) est constituée de PTFE.

6. Robinetterie sanitaire selon l'une des revendications 2 à 4, **caractérisée en ce que** la couche laissant passer le rayonnement (41) est constituée de TiO₂.

7. Robinetterie sanitaire selon l'une des revendications 2 à 4, **caractérisée en ce que** la couche laissant passer le rayonnement (41) est constituée d'un polymère hybride inorganique-organique.

8. Robinetterie sanitaire selon la revendication 7, **caractérisée en ce que** le polymère hybride inorganique-organique contient un réseau de silicate comme composant inorganique.
